# EUROPEAN PATENT APPLICATION

(11) **EP 1 508 341 A1**
(43) Date of publication of application: **23.02.2005**
(21) Application number: 04255052.5
(22) Date of filing: 20.08.2004
(51) Int. Cl.: A61L 2/22, A61L 2/20

(54) **Mist sterilization method**

(30) Priority: 22.08.2003 US 646296
(71) Applicant: ETHICON, INC., Somerville, NJ 08876 (US)
(72) Inventor: Lin, Szu-Min, Laguna Hill, California 92653 (US); Lukasik, Robert, Lake Elsinore, California 92530 (US)
(74) Representative: Fisher, Adrian John

(57) **Abstract**

A sterilization system employs a mist of chemical sterilant to sterilize devices such as medical instruments. A partial vacuum enhances dispersion of the mist into a sterilization chamber.

## Description

### Field of the Invention

The present invention relates to sterilization, and more particularly to sterilization employing a chemical sterilant in mist form.

### Background of the Invention

It has been recognized that a mist of chemical sterilant can effectively sterilize instruments, such as medical instruments. Vapor phase sterilization systems are also known, but require additional expense and complexity to produce and accommodate the deep vacuum and elevated temperatures associated with such systems.

One problem associated with delivery of a mist in a sterilization system is to adequately move the mist to fill the sterilization chamber and cover the item to be sterilized. Kodera et al., in U.S. Patent No. 4,366,125, provide the mist in extremely fine droplets to encourage its easy dispersion, and flow is enhanced with a carrier agent, namely warm air. Blidschun et al., in U.S. Patent No. 4,680,163, additionally encourage movement of the mist towards the device by inducing opposite electrical charges between the device and the mist. Sheiman, in U.S. Patent No. 6,379,616, attempt to use kinetic energy to flow the mist without a carrier. Each of these prior attempts rely upon positive pressure to push the mist into the sterilization chamber.

### Summary of the Invention

The present invention improves significantly over the prior attempts to move the mist efficiently to fill a sterilization chamber and cover the surface of a device therein to be sterilized.

A method of disinfecting or sterilizing an article according to the present invention comprises the steps of: placing the article into a chamber; reducing pressure in the chamber to a first pressure; introducing a mist comprising a sterilant into the chamber; and diffusing the mist through the chamber into contact with the article. The first pressure is below atmospheric pressure and above the vapor pressure of the sterilant thus enhancing diffusion of the mist throughout the chamber.

The method can employ many different sterilants which might work in mist form, with one preferable sterilant comprising hydrogen peroxide, such as a solution comprising hydrogen peroxide and water.

The first pressure is preferably at least 5 torr below atmospheric pressure, more preferably 15 torr below atmospheric pressure, and most preferably at least 30 torr below atmospheric pressure.

Preferably the article is sterilized in this procedure. Mere disinfection may suffice for many uses. Preferably, the procedure is sufficiently efficacious to sterilize a stainless steel blade with at least 10⁶ Bacillus stearothermophilus spores in less than 60 minutes.

Preferably, the chamber has an interior and the method further comprises sterilizing the interior of chamber.

Preferably, residual sterilant is removed from the chamber.

### Brief Description of the Drawings

FIG. 1 is a block diagram of a simple sterilization system according to the present invention;
FIG. 2 is a test chamber showing the efficacy of the mist delivery system according to the present invention;
FIG. 3 is a block diagram of a different embodiment of a sterilization system according to the present invention which employs a detachable container;
FIG. 4 is a front elevation view of an interface on a container in the system of FIG. 3, shown in an open position;
FIG. 5 is a front elevation view of the interface of FIG. 4 shown in a closed position;
FIG. 6 is a front elevation view of an alternative interface for the container of FIG. 3;
FIG. 7 is a cut-away view of an insert for the interface of FIG. 6 and having a self-closing mechanism shown in the closed position;
FIG. 8 is a cut-away view of the insert of FIG. 7, shown in the open position;
FIG. 9 is a cut-away view of an alternative insert for the interface of FIG. 6 and having a self-closing mechanism, shown in the closed position;
FIG. 10 is a cut-away view of the insert of FIG. 9, shown in the open position;
FIG. 11 is a cut-away view of an alternative embodiment of a sterilization container useful in the system of FIG.3; and
FIG. 12 is a cut-away view of a further alternative embodiment of a sterilization container useful in the system of FIG. 3.

### Detailed Description

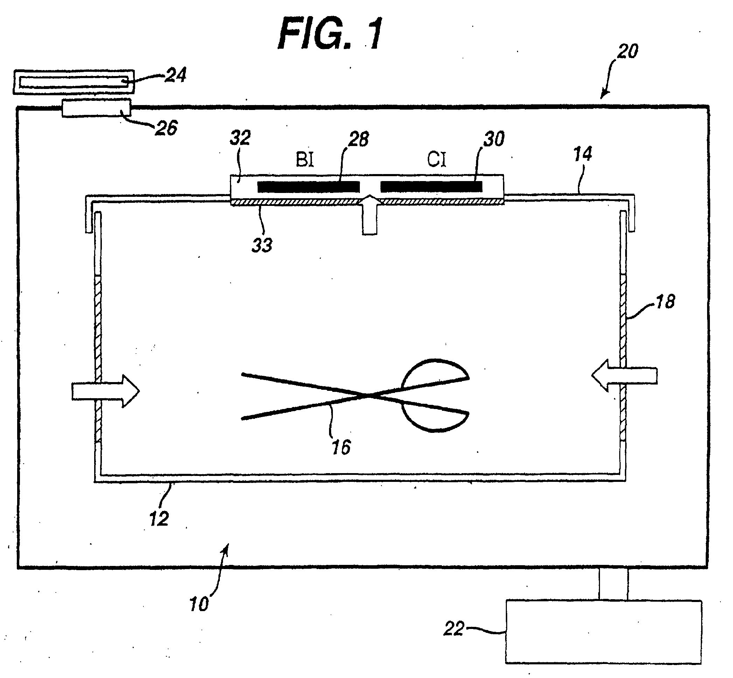

FIG. 1 discloses a sterilization container 10 comprising an enclosure 12 having a lid 14 and containing an instrument 16 to be sterilized. Ports 18 allow a sterilizing mist, such as a hydrogen peroxide solution mist, to enter the enclosure 12 and contact the instrument 16. The container 10 is enclosed within a sterilization chamber 20 that comprises a pump 22 for drawing at least a partial vacuum on the chamber 20 and a source 24 liquid sterilant. A mist generating apparatus 26 generates a mist from the sterilant and admits the mist into the chamber 20. Prior to such admission, the pump 22 draws a slight vacuum upon the chamber to induce suitable dispersion of the mist within the chamber 20.

For generating a mist of hydrogen peroxide, ultrasonic mist generators are preferred as they do not tend to decompose hydrogen peroxide. Such generators are employed in cold humidifiers. One suitable example is described by Takahashi et al. in U.S. Patent No. 5,299,739, incorporated herein by reference.

A biological indicator 28 and chemical indicator 30 are contained within a compartment 32, which is in fluid communication only through the enclosure 12, through a screen 33, to ensure adequate exposure to the sterilant mist and proper sterilization. A biological indicator indicates whether a test microorganism has been successfully killed in the sterilization process and a chemical indicator indicates the presence of, and in some instances and integrated exposure to, the sterilization media. Examples of biological and chemical indicators can be found in U.S. Patent Nos. 5,552,320, 5,942,438, 6,218,189, and 6,436,659 each of which is incorporated herein by reference.

As described above, prior system designers have sought more efficient means for delivering a sterilizing agent as a mist, essentially by forcing the mist into a chamber. The present invention dramatically improves over these systems by drawing the mist into the sterilization chamber 20 via a partial vacuum.

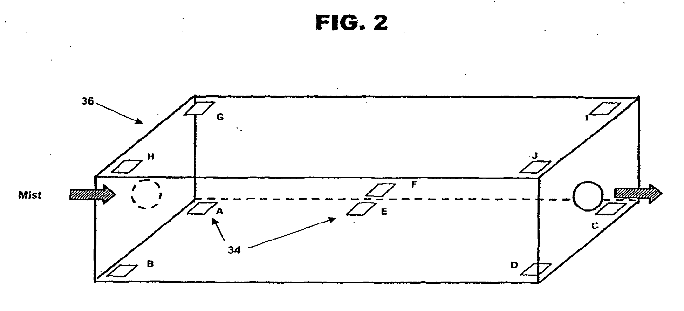

Experiments were conducted with 30% peroxide mist using either a 5 torr positive pressure to push or a 5 torr negative pressure to pull the mist into the chamber to determine the effect of mist uniformity on efficacy. Stainless steel blades 34 inoculated with 1.2x10⁶ Bacillus stearothermophilus spores were place at the corners and in the center of a chamber 36 (see FIG. 2). The results as shown in Table 1 indicate that the use of a reduced pressure to pull the mist produces better sterilization efficacy in the chamber 36 than the use of positive pressure to push the mist. Not all samples were sterilized after ten minutes with the use of 5 torr positive pressure to push mist into chamber 36. Most of the positive samples are located near the top of the chamber 36. In contrast, sterilization was achieved with no positive samples in 5 minutes with the use of 5 torr negative pressure to pull mist into chamber.

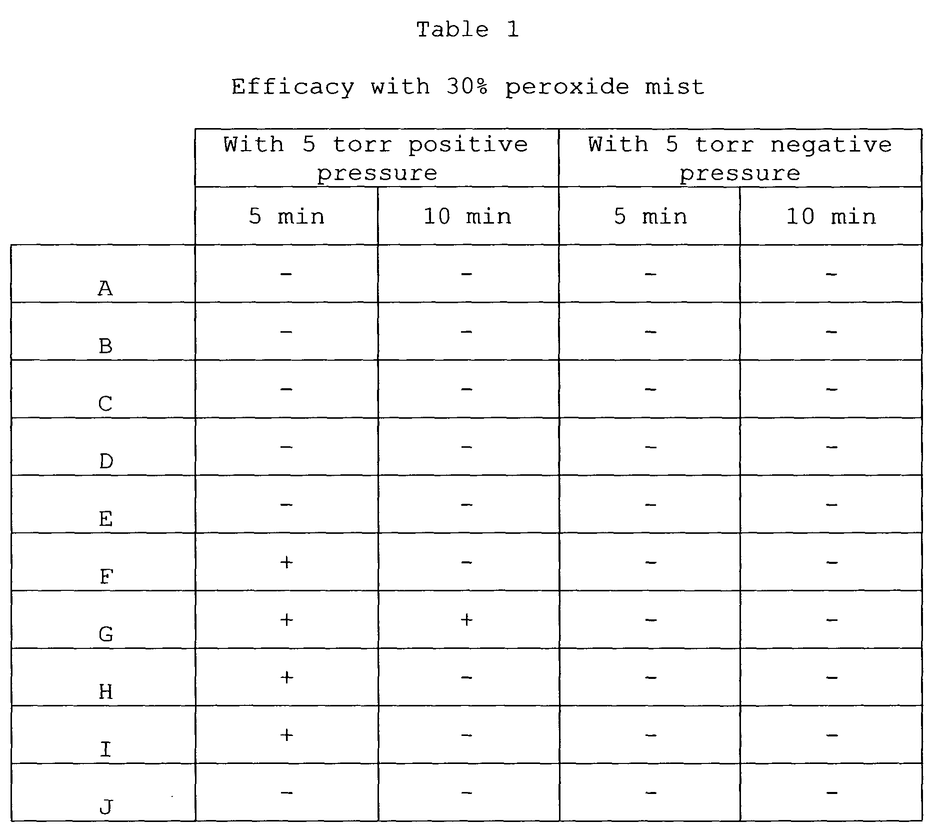

While a pressure of negative 5 torr was tested, other pressures, particularly lower pressures will likely enhance the results. With a sufficiently low pressure the mist will vaporize. Generally this enhances sterilization efficiency, but the pump necessary to achieve such pressure will be more complex and expensive than one employed solely to enhance dispersion of the mist within the container.

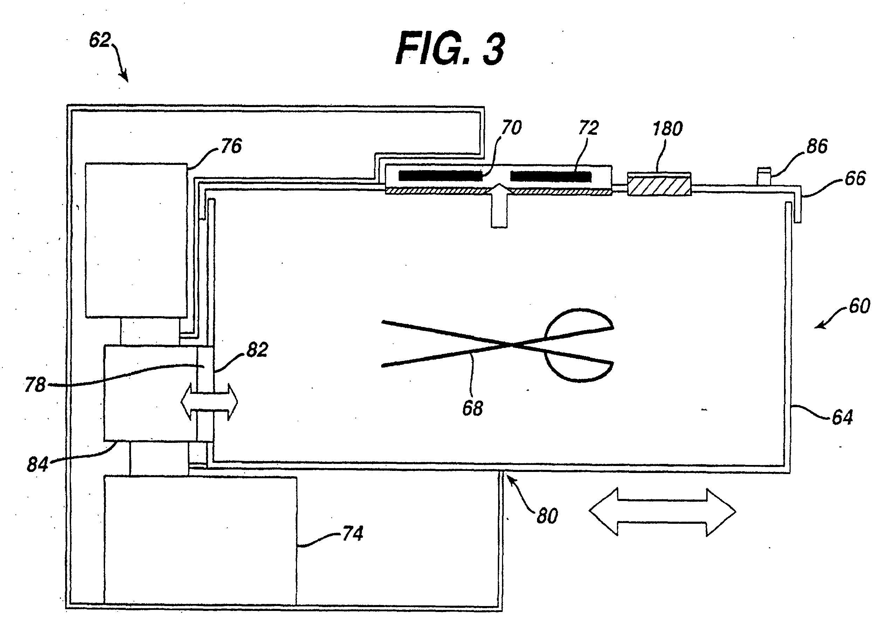

FIG. 3 illustrates an alternative sterilization system 62 employing a container 60 which comprises an enclosure 64 having a lid 66 and containing an instrument 68. The container 60 is preferably formed so as to be usable in other sterilizing systems such a steam, ethylene oxide or vapor phase hydrogen peroxide, thus simplifying user inventory. Therefore it is preferably formed of a material suitable for use in steam, hydrogen peroxide and ethylene oxide sterilization process, such as a liquid crystal polymer as described by Wu in U.S. Patent No. 6,379,631, incorporated herein by reference. Suitable polymers include polybenzoate-naphthalate; polybenzoate-terphthalate-bisphenol-isophthalate; polybenzoate-terphthalate-ethylene glycol; and polynaphthalate-amino terephthalate. A biological indicator 70 and chemical indicator 72 are provided as in the previous container.

The sterilizer 62 comprises a vacuum pump 74 and a sterilant source and mist generator 76 which connect via an interface 78 to the container 60. The sterilizer 62 has a receiving bay 80 for receiving a portion of the container 60. An interface 82 on the container 60 interfaces with the interface 78 on the sterilizer 62 to place the container enclosure 64 into fluid communication with the vacuum pump 74 and mist generator 76. One or more valves 84 controls the fluid communication between the mist generator 76 and the interface 78 and also the vacuum pump 74 and the interface 78. A simple sterilization process would involve engaging the container 60 into the receiving bay 80 of the sterilizer 62 and then drawing a slight vacuum on the enclosure 64 via the vacuum pump 74. Once the vacuum is established, mist from the mist generator 76 can be admitted into the enclosure 64 and dispersed throughout. After a sufficient period of time the sterilant will effect the sterilization of the instrument 68 and the container 60 can be removed from the receiving bay 80.

Depending upon the form of the interface 82, the container 60 may be left under vacuum after removal from the bay 80. Different formats for the interface will be described hereinafter. A vacuum relief valve 86 is provided and when the operator opens the vacuum relief valve 86 and hears an inrush of air the operator will know that the integrity of the container 60 has not been violated since the time of the sterilization procedure.

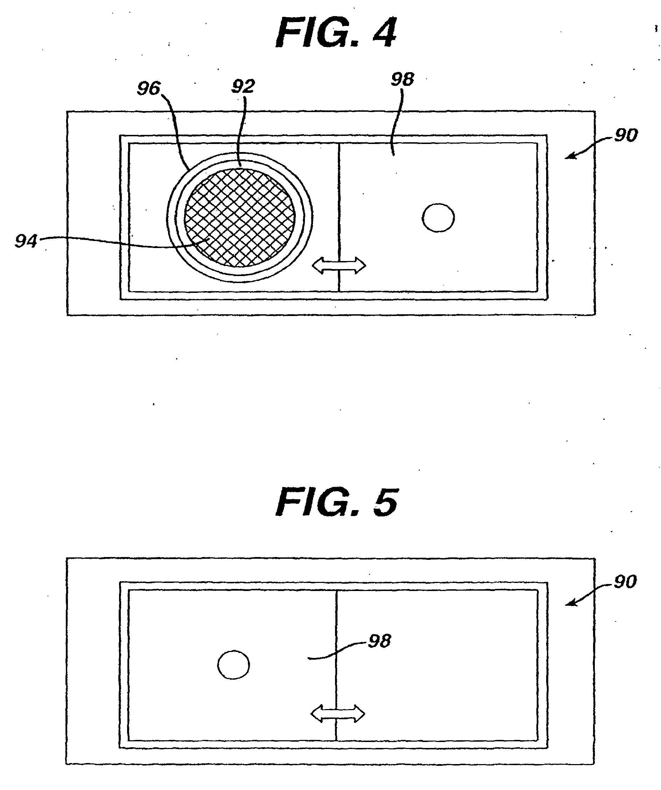

Turning also to FIGS. 4 and 5, an interface 90 comprises an aperture 92 into the enclosure 64, the aperture 92 being covered by a semipermeable filter 94 to allow passage of sterilizing media yet disallow passage of potentially contaminating microorganisms. This would provide flexibility in using the container for other sterilization systems such as a steam or vapor phase chemical sterilization type systems. An O-ring or gasket 96 surrounds the aperture 92 to help ensure a pressure tight seal with the interface 78 on the sterilizer 62. To close the aperture 92 for use in a self-contained sterilization procedure, a panel 98 slides over the aperture 92 and seals against the O-ring 96, as depicted in FIG. 5. For use in the present system 62 of FIG. 3, the semipermeable filter can be replaced with a screen. When used with a screen closing the panel 98, preferably automatically, prior to disconnection from the interface 78 allows storage of sterilized instruments within the container 60.

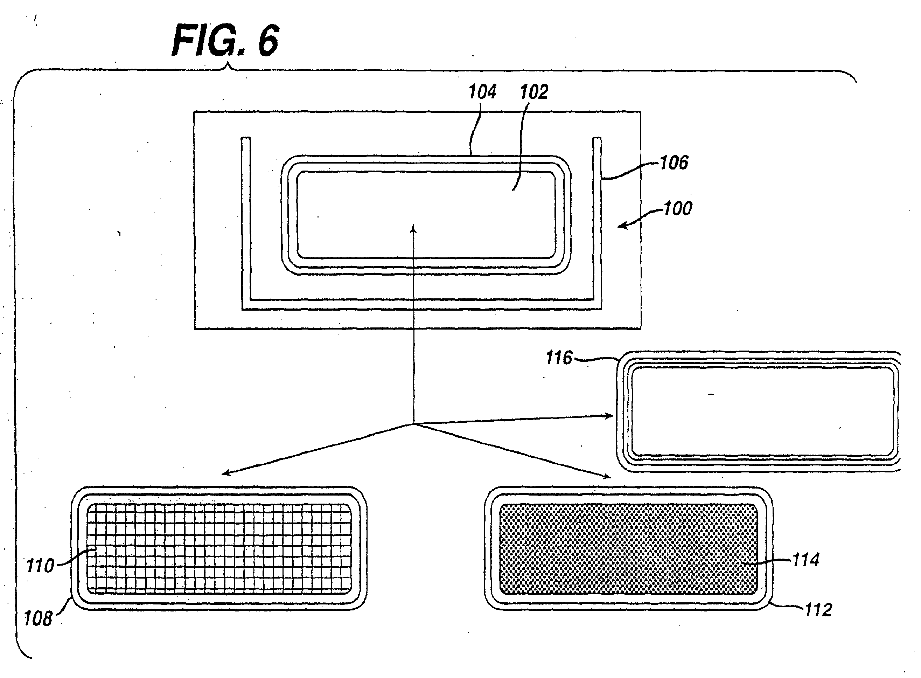

Turning also to FIG. 6, an alternative embodiment of an interface 100 comprises an aperture 102 surrounded by an O-ring or gasket 104 and having a frame 106 to receive various inserts. A first insert 108 fits into the frame 106 and comprises a screen 110 having holes large enough to easily pass a mist of sterilant media into the enclosure 64. A second alternative insert 112 comprises a semipermeable filter 114 for passing vapor phase sterilant media yet disallowing passage of contaminating microorganisms. A third insert 116 comprises merely a solid plate to block the aperture 102 entirely.

The first insert 108 having the screen 110 would be most useful for use with a sterilizer such as the sterilizer 62 in which the sterilant media enters the container 60 as a mist and in which the instruments 68 will not be stored in the container 60 after the procedure but rather will be used immediately thereafter, or where such instruments do not require complete sterility after the procedure. For instance, if the instruments 68 are dental instruments, a high level of sterilization efficiency may be desirable to kill difficult pathogens from a prior patient, but after the sterilization it would be acceptable to store the instruments in a clean environment yet not in a bacteria proof enclosure. Some means, such as insertion of a plate 116 to seal the container 60 would allow sterile storage therein.

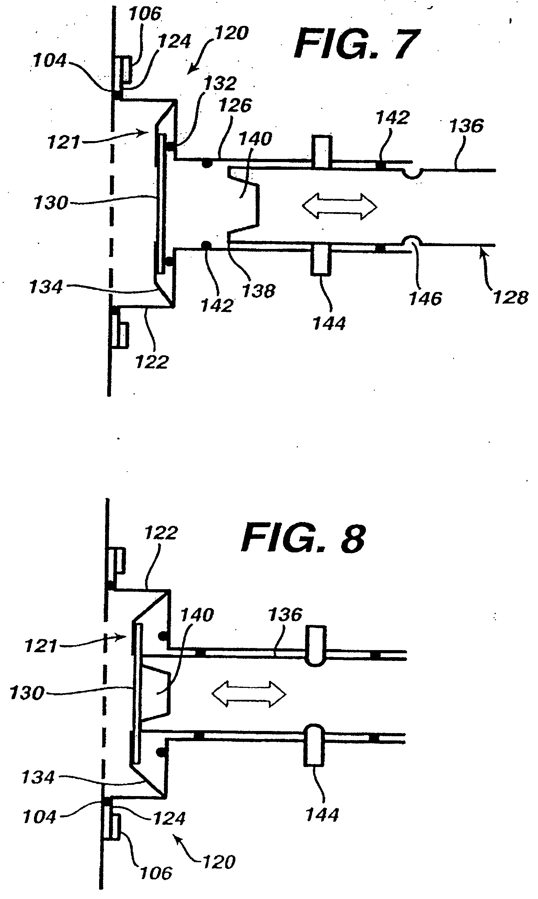

Turning also to FIGS. 7 and 8, an alternative insert 120 fits into the frame 106 and is primarily useful when employing a mist form of the sterilization media in the sterilizer 62 combined with the need to store the instruments 68 in the container 60 in a sterile form after the sterilization process is complete. The insert 120 employs a self-closing mechanism 121 which opens upon insertation of the container 60 into the sterilizer 62 to allow introduction of mist into the enclosure 64 and which closes automatically upon removal of the sterilization container 60 from the sterilizer 62 to seal the enclosure 64 from potentially contaminating microorganisms. The insert 120 comprises a body 122 having a flange 124 which fits into the frame 106 and seals against the O-ring or gasket 104. An open tube 126 extends outwardly from the body 122 to receive an adapter 128 from the sterilizer interface 78. The body 122 contains the self-closing mechanism 121. It comprises a valve member 130 biased toward a valve seat 132 on the body by a spring 134 or other biasing member. When seated on the valve seat 132 the valve member 130 seals the body 122 from the tube 126, thus effectively sealing the container aperture 102.

The adapter 128 comprises a pipe 136 having a distal end 138 that abuts the valve member 130 driving it away from the valve seat 132. One or more openings 140 of some form at or near the pipe distal end 138 place the pipe 136 into fluid communication with the body 122 and thus with the enclosure 64. Seals 142 provide a tight seal between the pipe 136 and tube 126. Spring loaded members 144 engage detents 146 on the pipe 136 to hold it in place. Upon removal of the container 60 from the bay 80 the pipe 136 will disengage from the valve member 130 and close the self-closing mechanism 121.

The mechanism of FIG. 7 relies upon the enclosure 64 to be near or above ambient pressure to keep the self-closing mechanism 121 closed. With a pressure slightly above ambient, opening of the relief valve 84 (FIG. 3) still causes an audible air rush to alert a user to the integrity of the container's seal. The slightly positive pressure further inhibits ingress of potentially contaminating microorganisms. In such a procedure, after the mist effects sterilization of the instrument or instruments 68, the pump 74 provides sterile, filtered air to the enclosure 64.

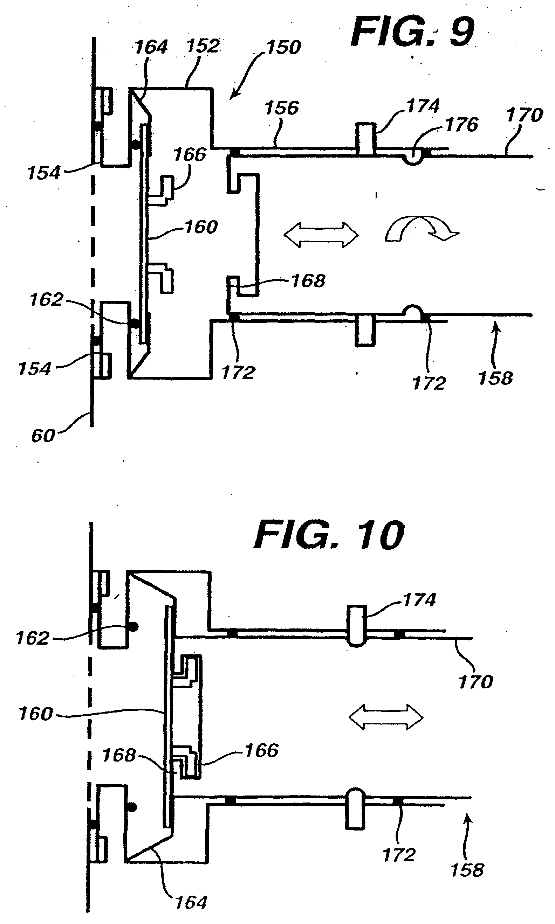

Turning to FIGS. 9 and 10, if a vacuum is desired for storage of the container 60, an alternative insert 150 may be used. It comprises a body 152 and flange 154 for connection to the container 60 and a tube 156 for receiving an adapter 158 from the sterilizer 62 (not shown in FIGS. 9 and 10). The body 152 contains a valve member 160 biased toward a valve seat 162 by a biasing member 164. However, it differs from the previous embodiment in that it closes toward the container 60, such that a vacuum in the container 60 holds the valve member 160 closed. A flange 166 on the valve member 160 engages a flange 168 on a pipe 170 of the adapter 158. The pipe 170 rotates to engage the flanges 166 and 168 with each other and is then retracted slightly to pull the valve member 160 away from the valve seat 162. Seals 172 are provided between the pipe 170 and tube 156. Spring loaded members 174 engage detents 176 on the pipe 170 to hold the parts in the proper orientation. After the sterilization process the process is reversed to seat the valve member 160.

While the provision of various inserts 108, 112, 116, 120 and 150 provides the most flexibility, either of the inserts 120 or 150 could be integral with the container rather than removable.

Further, to enhance its flexibility, the container 60 may include multiples of the features disclosed herein. For instance, especially when configured with the inserts 120 and 150 with their self-closing feature it is preferred to have, especially at the top and bottom of the container 60 (best seen in FIG. 3), additional filtered ports 180 which may be sealed. Such ports could be similar to the interface 90, or could have screw-on covers or other sealing mechanisms. Preferably, the filters can be replaced, as is preferable with any of the filters discussed herein. Such additional ports provide enhanced diffusion of sterilant into and out of the container 60 when used in a standard chamber 20 such as disclosed in FIG. 1. When used in the process of FIG. 3 the ports 180 would be left closed.

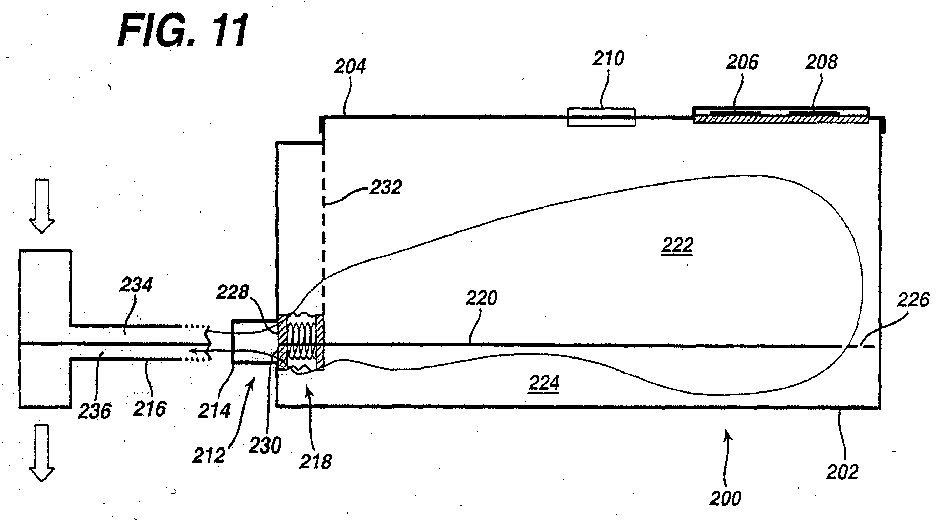

FIG. 11 discloses an alternative embodiment of a container 200, especially useful in the sterilizer 62 of FIG. 3, but offering enhanced flow through the container 200. It comprises an enclosure 202 having a lid 204, provision for a biological indicator 206 and chemical indicator 208 and an optimal sealable filtered port 210 in the lid 204. The port 210, and perhaps addition ports in the bottom or other surfaces, allow the container to also function in traditional steam and chemical vapor sterilization systems. An inlet/exit port 212 has an opening 214 for receiving a probe 216 from the sterilizer 60. A normally closed spring loaded valve 218 opens when the probe 216 is inserted into the opening 214 and abuts the valve 218. A solid partition 220 separates the enclosure 202 into an upper portion 222 and lower portion 224. A screened aperture 226 in the partition, away from the port 212, connects the upper and lower portions 222 and 224. The opening 214 is partitioned into an upper path 228 and lower path 230. A screened partition 232 separates the upper portion 222 from the upper path 228 and the lower path 230 communicates with the lower portion 224. The probe 216 is preferably similarly separated into an upper path 234 adapted to communicate with the opening upper path 228 and a lower path 236 adapted to communicate with the port lower path 230.

When the probe 216 enters the opening 214 it opens the valve 218. The vacuum pump 74 draws a partial vacuum on the container 200 and then sterilizing mists flow in from the probe upper path 234 into the opening upper path 228 and into the enclosure upper portion 222. The mist can later exit the enclosure 202 by flowing through the aperture 226 into the enclosure lower portion 224 and out through the opening and probe lower paths 230 and 236 under the draw of the vacuum pump 74. After the probe 216 is removed, the spring loaded valve 218 closes and seals the enclosure 202. Instruments for sterilization, which were placed into the upper portion 222 through the lid 204, and are now sterile.

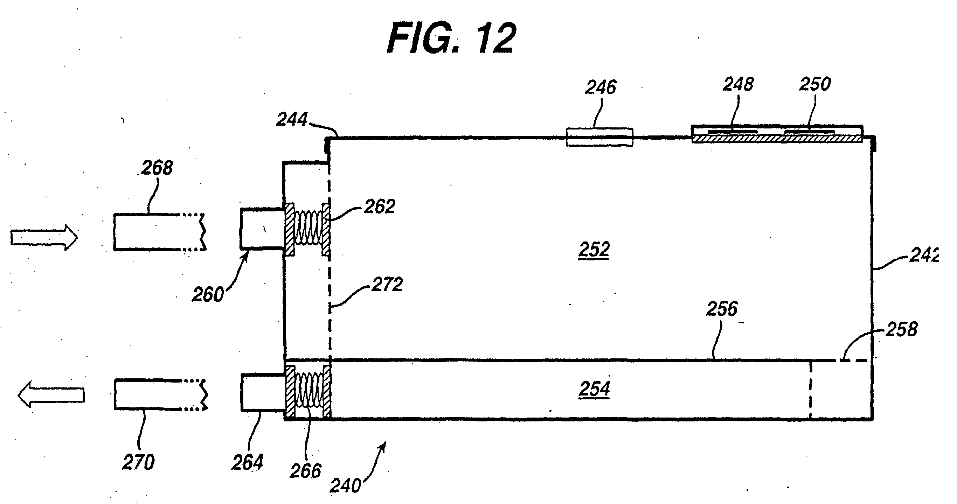

FIG. 12 illustrates a similar container 240 comprising an enclosure 242 having a lid 244, a filtered port 246 and provisions for a biological indicator 248 and chemical indicator 250. The enclosure 242 is separated into an upper portion 252 and lower portion 254 by a solid partition 256 having a screened aperture 258. The container 240 has an inlet port 260 into the upper portion 252, with a spring-loaded valve 262 and an exit port 264 from the lower portion 254, also with a spring-loaded valve 266. The inlet port 260 receives an inlet probe 268 and the exit port 264 receives an exit probe 270. A screen 272 separates the inlet port 260 from the upper portion 252. Mists flow from the inlet probe 268 into the enclosure upper portion 252 and exit from the exit probe 270 as in the previous embodiment.

The flow could be continuous, in which case it would be desirable to continually recirculate the same sterilant through the enclosure 242. Alternatively, the exit probe 270 can be used to lower the pressure in the enclosure 242 to and then the inlet probe 268 can supply sterilant mist such as hydrogen peroxide mist to the enclosure 242. After a sufficient time to effect sterilization, the exit probe 270 can draw out the sterilant.

One of skill in the art will recognize that the location of the ports 260 and 264 can be changed to address other functional needs while keeping with the concept of flowing gases more efficiently through the container 240. For instance, they could be located on the bottom of the container with suitable partitioning within the enclosure 242 to route incoming gases to the enclosure upper portion 252. Rather than have spring-loaded valve 262 and 266 which move directly away from the incoming probes 268 and 270, spring-loaded flap valves (not shown) which rotate away from the incoming probe could be substituted therefor and would not tend to push the probe out after its insertion.

To remove residual sterilant, especially hydrogen peroxide, it may be advisable to circulate warm dry air through any of the containers disclosed above, to draw a vacuum with the vacuum pump or to induce a plasma such as in the Jacobs et al. U.S. Patent No. 4,643,876, incorporated herein by reference.

While the invention has been particularly described in connection with specific embodiments thereof, it is to be understood that this is by way of illustration and not of limitation, and that the scope of the appended claims should be construed as broadly as the prior art will permit.

## Claims

1. A method of disinfecting or sterilizing an article comprising the steps of:
placing the article into a chamber;
reducing pressure in the chamber to a first pressure;
introducing a mist comprising a sterilant into the chamber;
diffusing the mist through the chamber into contact with the article; and
wherein the first pressure is below atmospheric pressure and above the vapor pressure of the sterilant whereby to enhance diffusion of the mist throughout the chamber.

2. A method according to claim 1 wherein the sterilant comprises hydrogen peroxide.

3. A method according to claim 2 wherein the mist comprises a solution comprising hydrogen peroxide and water.

4. A method according to claim 1 wherein the first pressure is at least 5 torr below atmospheric pressure.

5. A method according to claim 4 wherein the first pressure is at least 15 torr below atmospheric pressure.

6. A method according to claim 4 wherein the first pressure is at least 30 torr below atmospheric pressure.

7. A method according to claim 1 further comprising sterilizing the article.

8. A method according to claim 7 sufficiently efficacious to sterilize a stainless steel blade with at least 10⁶ Bacillus stearothermophilus spores in less than 60 minutes.

9. A method according to claim 1 wherein the chamber has an interior and the method further comprising sterilizing the interior of chamber.

10. A method according to claim 1 further comprising removing residual sterilant from the chamber.
